# FASCICULE DE BREVET EUROPEEN

(11) **EP 2 396 400 B1**
(45) Date de publication et mention de la délivrance du brevet: **22.04.2015**
(21) Numéro de dépôt: 09784359.3
(22) Date de dépôt: 12.02.2009
(51) Int. Cl.: C12N 1/12, C12R 1/89, A23K 1/18, A23K 1/00

(54) **PROCEDE DE CULTURE D'ALGUES UNICELLULAIRES, MILIEU DE CULTURE ET UTILISATIONS**
VERFAHREN ZUR KULTIVIERUNG VON UNIZELLULÄREN ALGEN, KULTUR-MEDIUM UND VERWENDUNGEN
METHOD FOR GROWING UNICELLULAR ALGAE, GROWING MEDIUM AND USES

(43) Date de publication de la demande: 21.12.2011
(73) Titulaire: Fermentalg, 33500 Libourne (FR)
(72) Inventeur: CALLEJA, Pierre, F-33500 Libourne (FR)
(74) Mandataire: Santarelli
(86) Numéro de dépôt international: PCT/FR2009/050221
(87) Numéro de publication internationale: WO 2010/092243

(56) Documents cités:
- EP-A- 1 142 985
- FR-A- 2 923 219
- WAGNER F W ET AL: "Oxygen-tolerant strain of chlorella sorokiniana." APPLIED MICROBIOLOGY JAN 1969, vol. 17, no. 1, janvier 1969 (1969-01), pages 139-144, XP002551170 ISSN: 0003-6919
- VELA G R ET AL: "On the nature of mixed cultures of Chlorella pyrenoidosa TX 71105 and various bacteria." JOURNAL OF GENERAL MICROBIOLOGY JAN 1966, vol. 42, no. 1, janvier 1966 (1966-01), pages 123-131, XP002551171 ISSN: 0022-1287
- THERIAULT R J: "HETEROTROPHIC GROWTH AND PRODUCTION OF XANTHOPHYLLS BY CHLORELLA PYRENOIDOSA." APPLIED MICROBIOLOGY MAY 1965, vol. 13, mai 1965 (1965-05), pages 402-416, XP002551172 ISSN: 0003-6919

## Description

La présente invention se rapporte à un procédé de culture d'algues unicellulaires à partir d'une souche de *Chlorella sorokiniana* et à un milieu de culture.

L'invention se rapporte également à l'utilisation des algues cultivées par ce procédé, en particulier comme nourriture pour rotifères, ainsi qu'aux aliments pour pisciculture les incluant.

Les algues sont couramment utilisées en matière d'aquaculture et d'aquariophilie. Elles constituent notamment une part importante de la nourriture des rotifères, qui sont ensuite utilisées pour l'élevage des larves de poisson.

Toutefois, toutes les algues ne sont pas satisfaisantes et ne sont pas adaptées à l'élevage de tous poissons. Ces algues doivent en effet répondre à certains critères. En particulier elles doivent être de petite taille, résister au choc de salinité pour les poissons d'eau de mer, et permettre d'équilibrer le milieu d'élevage.

On sait que les chlorophycées unicellulaires de types chlorelles, sont utilisées comme nourriture pour les rotifères destinées à la pisciculture.

Les chlorelles sont des organismes photosynthétiques autotrophes. Elles peuvent être cultivées en plein air par croissance autotrophe. Elles peuvent également être cultivées par croissance hétérotrophe ou mixotrophe en cuve par fermentation et en présence de matières organiques.

Différentes méthodes de culture de chlorelles ont été décrites, telles que dans les demandes de brevet GB-1.109.659 et GB-1.109.658.

D1 [Ref 01] divulgue la culture des souches de *Chlorella sorokiniana* dans le milieu de Knop et 0,5 % de glucose avec une haute concentration en oxygène pour sélectionner des souches pouvant tolérer l'oxygène.

Toutefois, ces méthodes ne permettent pas d'obtenir de bons rendements. Les concentrations en algues sont très basses, inférieures à 8 g/L, et les temps de génération sont très élevés.

En outre, les souches de chlorelle utilisées ne sont pas adaptées à tous les types de poissons.

Il subsiste donc un besoin pour un procédé de culture de microalgues adaptées à une utilisation pour l'élevage de poissons, permettant une bonne croissance des microalgues, c'est à dire avec un temps de latence et un temps de génération très faibles, ainsi qu'une concentration en biomasse et des rendements élevés.

C'est ce à quoi répond la présente invention en proposant un procédé de culture d'algues unicellulaires à partir d'une souche de *Chlorella sorokiniana,* en hétérotrophie ou mixotrophie par fermentation, dans un milieu de culture comprenant entre 4 et 6 g/L de glucose et entre 120 et 190 mg/L de calcium.

Les algues unicellulaires obtenues peuvent être utilisées en tant que nourriture pour les rotifères destinés à la pisciculture. Elles sont particulièrement adaptées pour l'élevage du bar et de la daurade.

Selon un autre aspect, l'invention vise également un milieu de culture d'algues unicellulaires à partir d'une souche de *Chlorella sorokiniana,* comprenant entre 4 et 6 g/L de glucose et entre 120 et 190 mg/L de calcium.

Enfin, l'invention vise aussi les aliments pour la pisciculture composés d'au moins une algue unicellulaire obtenue par le procédé de culture selon l'invention. Les algues unicellulaires obtenues selon l'invention peuvent également être utilisées en tant qu'usines cellulaires, pour extraire des molécules et produire de la biomasse, par exemple des molécules destinées à la pharmacie ou la cosmétologie.

L'invention est maintenant décrite en détails.

L'invention vise un procédé de culture d'algues unicellulaires à partir d'une souche de *Chlorella sorokiniana.*

Cette souche est particulièrement adaptée à la production de rotifères et à l'élevage de poissons. En outre, il s'agit d'une souche d'eau douce résistante à la salinité, donc supportant l'eau de mer, et présentant une croissance rapide en hétérotrophie sur glucose.

Le procédé de culture est réalisé en hétérotrophie ou mixotrophie par fermentation dans un milieu de culture comprenant notamment du glucose et du calcium. Le milieu de culture peut contenir d'autres éléments, tels que du potassium, du magnésium, des micro-éléments et des vitamines.

Les concentrations en glucose et en calcium du milieu de culture ont une grande influence sur la croissance des algues unicellulaires, en particulier sur le temps de latence, le temps de génération, ainsi que sur la concentration en biomasse et le rendement.

Selon un aspect de l'invention, le milieu de culture doit contenir une quantité de glucose suffisante pour la fermentation, mais pas trop élevée pour éviter d'inhiber la croissance des algues. En particulier, le milieu de culture selon l'invention présente une concentration en glucose comprise entre 4 et 6 g/L.

Un mode réalisation préféré consiste à utiliser la technique de culture en Fed- Batch, qui permet de maintenir le glucose à des concentrations non inhibitrices tout en accumulant de la biomasse.

Préférentiellement, le glucose utilisé dans le milieu de culture est du D-glucose ou dextrose, en particulier du dextrose provenant de la biotransformation de l'amidon, par exemple à partir de maïs, de blé ou de pomme de terre. En effet, les hydrolysats de l'amidon sont des molécules de petite taille, qui sont plus facilement assimilables par les algues qu'un autre sucre et qui permettent un bien meilleur développement de la biomasse.

Selon un autre aspect de l'invention, la croissance de la souche de *Chlorella sorokiniana* dépend de la présence dans le milieu de culture, de calcium en forte concentration, à savoir entre 120 et 190 mg/L.

Selon un mode de réalisation particulièrement adapté, le milieu de culture comprend 5 g/L de glucose et 150 g/L de calcium.

Avantageusement, un tel milieu permet d'obtenir un temps de latence de 29,4 heures, et un temps de génération de 4,6 heures.

Le procédé de culture selon l'invention doit être réalisé à une température moyenne permettant une bonne croissance des algues, préférentiellement comprise entre 30 et 32°C.

Un exemple non limitatif de milieu de culture adapté au procédé de culture selon la présente invention, est décrit dans le tableau suivant:

| | | |
|---|---|---|
| Macro-éléments | Dextrose | 5 g/L |
| | KN03 | 1,5 g/L |
| | KH2P04 | 0,2 g/L |
| | MgS04,7 H20 | 0,6 g/L |
| | CaCI2,2H2O | 0,48 g/L |
| Micro-éléments (Solution stock concentrée 1000 fois) | Na2EDTA,2H2O | 4,7730 mg/L |
| | FeS04,7H2O | 0,6919 mg/L |
| | H3B03 | 3,5376 mg/L |
| | ZnS04,7H2O | 1,2641 mg/L |
| | MnC12,4H20 | 0,0505 mg/L |
| | Na2Mo04, 2H2O | 0,3050 mg/L |
| | CuS04,5H2O | 0,0785 mg/L |
| | CoS04,5H2O | 0,0402 mg/L |
| Vitamines (Solution stock concentrée 1000 fois) | Biotine | 0,04 mg/L |
| | Thiamine | 0,04 mg/L |
| | Cyanocobalamine | 0,04 mg/L |

Avantageusement, le procédé de culture d'algues unicellulaires selon l'invention permet de réduire le temps de culture à moins de 40 heures, les temps de latence et de génération étant très faibles. En outre les rendements sont importants et la concentration en microalgues obtenue est comprise entre 60 et 70 g/L, ce qui est environ dix fois plus élevé que les concentrations obtenues avec les procédés de culture connus.

Les microalgues obtenues par le procédé de culture à partir de *Chlorella sorokiniono* selon l'invention, peuvent être utilisées en tant que fourrage pour la pisciculture. Elles sont particulièrement adaptées pour l'élevage du bar et de la daurade.

Elles peuvent être utilisées comme nourriture de proies vivantes de types rotifères, qui sont ensuite elles-mêmes utilisées pour nourrir des poissons. Elles peuvent également être directement dispersées dans des bassins d'élevage de poissons pour équilibrer le bassin.

Selon un autre aspect, elles peuvent aussi avantageusement être utilisées en tant qu'usines cellulaires, pour extraire des molécules et produire de la biomasse, par exemple des molécules destinées à la pharmacie ou la cosmétologie.

Enfin, l'invention vise également les aliments pour la pisciculture composés d'au moins une algue unicellulaire obtenue par le procédé de culture à partir de *Chlorellasorokiniana* selon l'invention.

## Revendications

1. Milieu de culture d'algues unicellulaires à partir d'une souche de *Chlorella sorokiniana* comprenant entre 120 et 190 mg/L de calcium et entre 4 et 6 g/l de glucose.

2. Milieu de culture d'algues unicellulaires selon l'une des précédentes revendications, **caractérisé en ce qu'**il comprend 5 g/L de glucose et 150 mg/L de calcium.

3. Milieu de culture d'algues unicellulaires selon l'une des précédentes revendications, **caractérisé en ce que** le glucose est du D-glucose ou dextrose.

4. Milieu de culture d'algues unicellulaires selon l'une des précédentes revendications, **caractérisé en ce que** le glucose est du D-glucose ou dextrose, provenant de la transformation de l'amidon.

5. Milieu de culture d'algues unicellulaires selon l'une des précédentes revendications, **caractérisé en ce que** la température est comprise entre 30 et 32°C.

6. Milieu de culture d'algues unicellulaires selon l'une des précédentes revendications, **caractérisé en ce que** le milieu de culture comprend également du potassium, du magnésium, des micro-éléments et des vitamines.

7. Procédé de culture d'algues unicellulaires à partir d'une souche de *Chlorella sorokiniana,* en hétérotrophie ou mixotrophie par fermentation dans un milieu de culture selon l'une des revendications 1 à 6.

## Patentansprüche

1. Kulturmedium für einzellige Algen ausgehend von einem Stamm der *Chlorella sorokiniana,* umfassend zwischen 120 und 190 mg/l Calcium und zwischen 4 und 6 g/l Glucose.

2. Kulturmedium für einzellige Algen nach einem der vorhergehenden Ansprüche, **dadurch gekennzeichnet, dass** es 5 g/l Glucose und 150 mg/l Calcium enthält.

3. Kulturmedium für einzellige Algen nach einem der vorhergehenden Ansprüche, **dadurch gekennzeichnet, dass** die Glucose D-Glucose oder Dextrose ist.

4. Kulturmedium für einzellige Algen nach einem der vorhergehenden Ansprüche, **dadurch gekennzeichnet, dass** die Glukose aus der Verarbeitung von Stärke herrührende D-Glucose oder Dextrose ist.

5. Kulturmedium für einzellige Algen nach einem der vorhergehenden Ansprüche, **dadurch gekennzeichnet, dass** die Temperatur zwischen 30 und 32 °C umfasst.

6. Kulturmedium für einzellige Algen nach einem der vorhergehenden Ansprüche, **dadurch gekennzeichnet, dass** das Kulturmedium auch Kalium, Magnesium, Spurenelemente und Vitamine umfasst.

7. Verfahren zur Kultivierung einzelliger Algen ausgehend von einem Stamm der *Chlorella sorokiniana,* heterotroph oder mixotroph, durch Fermentation in einem Kulturmedium nach einem der Ansprüche 1 bis 6.

## Claims

1. Medium for culturing unicellular algae from a strain of *Chlorella sorokiniana,* comprising between 120 and 190 mg/l calcium and between 4 and 6 g/l glucose.

2. Medium for culturing unicellular algae according to any of the preceding claims, **characterised in that** it comprises 5 g/l glucose and 150 mg/l calcium.

3. Medium for culturing unicellular algae according to any of the preceding claims, **characterised in that** the glucose is D-glucose or dextrose.

4. Medium for culturing unicellular algae according to any of the preceding claims, **characterised in that** the glucose is D-glucose or dextrose originating from the conversion of starch.

5. Medium for culturing unicellular algae according to any of the preceding claims, **characterised in that** the temperature is between 30 and 32 °C.

6. Medium for culturing unicellular algae according to any of the preceding claims, **characterised in that** the culture medium also comprises potassium, magnesium, trace elements and vitamins.

7. Method for culturing unicellular algae from a strain of *Chlorella sorokiniana* in heterotrophy or mixotrophy by fermentation in a culture medium according to any of claims 1 to 6.
